# EUROPÄISCHE PATENTSCHRIFT

(11) **EP 0 688 768 B1**
(45) Veröffentlichungstag und Bekanntmachung des Hinweises auf die Patenterteilung: **02.09.1998**
(21) Anmeldenummer: 94109605.9
(22) Anmeldetag: 22.06.1994
(51) Int. Cl.: C07D 223/22

(54) **Verfahren zur Herstellung von 5H-Dibenz/b,f/azepin-5-carboxamid**
Method for preparing 5-H-dibenz/b,f/azepin-5-carboxamid
Procédé pour la préparation de 5-H-dibenz-/b,f/azépin-5-carboxamide

(43) Veröffentlichungstag der Anmeldung: 27.12.1995
(73) Patentinhaber: ARZNEIMITTELWERK DRESDEN GmbH, FACHBEREICH PATENTE, D-01435 Radebeul (DE)
(72) Erfinder: Eckardt, Rudolf, Dr., D-01445 Radebeul (DE); Jänsch, Hans-Joachim, Dr., D-01445 Radebeul (DE)

(56) Entgegenhaltungen:
- EP-A- 0 029 409
- EP-A- 0 277 095
- EP-A- 0 423 679
- DE-A- 2 307 174
- DE-A- 2 637 666
- CHEMISTRY AND INDUSTRY 7. August 1965 Seiten 1428 - 1429 DURANT,G.J. 'An improved Synthesis of N,N,-Diarylureas'
- HOUBEN-WEYL 'Methoden der Organischen Chemie' 1952 , VERLAG G.THIEME , STUTTGART Seiten 157-159
- CHEMICAL ABSTRACTS, vol. 85, no. 3, 19. Juli 1976, Columbus, Ohio, US; abstract no. 21150u, VEB ARZNEIMITTELWERK '5-Substituted dibenz(b,f)azepines' Seite 671 ; & JP-A-74 126 689 (VEB ARZNEIMITTELWERK DRESDEN) 4. Dezember 1974

## Beschreibung

Die Erfindung betrifft ein Verfahren zur Herstellung von 5H-Dibenz/b,f/azepin-5-carboxamid. Dieser Stoff ist unter dem Freinamen Carbamazepin in die Therapie eingeführt. Er besitzt wertvolle physiologische Eigenschaften. Carbamazepin dient zur Behandlung einer Vielzahl von Erkrankungen des Zentralnervensystems.

Für die Synthese von Carbamazepin sind mehrere Verfahren bekannt.
So kann man Carbamazepin herstellen durch
- Umsetzung von Iminostilben mit Phosgen zu 5H-Dibenz/b,f/azepin-5-carbonsäurechlorid und nachfolgender Zugabe von Ammoniak zu der entweder isolierten Verbindung oder auch zum erhaltenen Reaktionsgemisch (US-PS 2 948 718, DD-PS 297 962 und 298 508 sowie EP 423 679) oder
- Umsetzung von 10,11-Dihydro-5H-dibenz/b,f/azepin nacheinander mit Phosgen, 1,3-Dibrom-5,5-dimethylhydantoin, Kaliumcarbonat und Ammoniak (GB-PS 1 245 606) oder
- Umsetzung von Iminostilben mit einem Acylisocyanat zu 5H-Dibenz/b,f/azepin-5-(N-acyl)-carboxamid und anschließender Hydrolyse (DE-OS 23 07 174) oder
- Umsetzung von 5-(Methylisothiocarbamoyl)-5H-dibenz/b,f/azepin-hydrojodid mit Alkalien (DE-OS 26 37 666) oder
- saure oder alkalische Hydrolyse von 5-Cyano-5H-Dibenz/b,f/azepin (EP 029 409).

Diese bekannten Verfahren besitzen eine Reihe von Nachteilen. So ist z.B. bei vielen der aufgeführten Verfahren der Einsatz des hochtoxischen Phosgens bzw. von Halogencyan erforderlich. Bei der Synthese gemäß DE-OS 23 07 174 werden die instabilen und schwer zugänglichen Acylisocyanate benötigt. Bei der Darstellung gemäß DE-OS 26 37 666 entsteht als Nebenprodukt das giftige Methylmercaptan.

Carbamazepin kann desweiteren hergestellt werden durch
- Umsetzung von Iminostilben mit Cyansäure in einem organischen Lösungsmittel oder Lösungsmittelgemisch in Gegenwart eines sauren Mittels (EP 277 095).
   Das saure Mittel nimmt an der Reaktion nur katalytisch teil und dient der Erhöhung der Umsatzgeschwindigkeit von Iminostilben und Cyansäure.
   Die für die Umsetzung erforderliche Cyansäure wird gasförmig in das Reaktionsgemisch eingeleitet, sie kann aber auch durch Säurebehandlung eines Salzes der Cyansäure gewonnen werden.
   Da die Cyansäure mit Wasser, Alkoholen und Aminen unerwünschte Nebenreaktionen eingeht, erfolgt die Umsetzung unter weitgehend aprotischen Bedingungen, d.h. in möglichst wasser-, alkohol- und aminfreier organischer Lösung und unter Wasserdampfauschluß.
Diese Bedingungen erschweren die gesamte Reaktionsführung und erfordern einen zusätzlichen materiellen und technischen Aufwand.

Der vorliegenden Erfindung liegt nun die Aufgabe zugrunde, ein technisch einfach handhabbares Verfahren zur Herstellung von Carbamazepin zu finden, das die vorgenannten Nachteile nicht aufweist.

Entsprechend der vorliegenden Erfindung wird das überraschend dadurch erreicht, daß man Iminostilben mit Alkalicyanaten in Essigsäure , Essigsäure-Wasser- oder Essigsäure-Alkohol-Gemischen umsetzt.

Die Umsetzung von Iminostilben mit einem Alkalicyanat, wie Natrium- oder Kaliumcyanat, kann im Temperaturbereich von 20 bis etwa 100°C durchgeführt werden.
Eine bevorzugte Ausführungsform des erfindungsgemäßen Verfahrens besteht darin, daß man bei 60°C zu einer gerührten Suspension von Iminostilben in Essigsäuregemischen das Alkalicyanat allmählich zugibt, wobei der Überschuß an Cyanat etwa 50 Molprozent, bezogen auf Iminostilben, beträgt.

Nach dem erfindungsgemäßen Verfahren kann der Wassergehalt des Essigsäure-Wasser-Gemisches bis zu 20 Gewichtsprozent betragen.
Erfolgt die erfindungsgemäße Umsetzung in einem Essigsäure-Alkohol-Gemisch, so können bis zu 10 Gewichtsprozent Alkohol enthalten sein.
Bevorzugt werden Alkohole, wie Methanol und Ethanol in diese Reaktion eingesetzt.

Die allmähliche Zugabe des Alkalicyanates kann durch portionsweise Zugabe des Feststoffes erfolgen . Vorteilhafter ist jedoch das Zutropfen einer wäßrigen Lösung des Alkalicyanates, insbesondere wenn das in Wasser leicht lösliche Kaliumcyanat verwendet wird .
Durch die Möglichkeit des Zutropfens einer wäßrigen Lösung eines Alkalicyanates kann das erfindungsgemäße Verfahren technisch besonders einfach durchgeführt werden.

Das für die Umsetzung von Iminostilben mit einem Alkalicyanat verwendete Lösungsmittelgemisch kann nach der Abdestillation aus dem Reaktionsgemisch für weitere erfindungsgemäße Umsetzungen eingesetzt werden.

Der Erfolg des erfindungsgemäßen Verfahrens war in mehrfacher Hinsicht überraschend.
Aus der Literatur ist zwar bekannt, daß eine Anzahl aromatischer primärer Amine in wäßrig-essigsaurer Lösung mit Alkalicyanaten zu den betreffenden Harnstoffderivaten umgesetzt werden können. Dabei wird beispielsweise bei Kurzer (Org. Synth. Coll. Vol. IV, S.49) in Essigsäurewasser-Gemischen im Verhältnis von 1 : 10 bis 1 : 1 gearbeitet.
Doch unter diesen Reaktionsbedingungen gelingt die Umsetzung von Iminostilben, einem sekundären Diarylamin, mit Alkalicyanaten nicht.
Zum einen weist Iminostilben eine viel geringere Basizität gegenüber den vorgenannten aromatischen primären Aminen auf und zum anderen besitzt Iminostilben in Essigsäure eine viel geringere Löslichkeit als diese Amine. Diese geringe Löslichkeit sinkt durch Wasser- als auch Alkohol-Zusatz weiter.

Nach den Darlegungen im EP 277 095 mußte der Fachmann sogar annehmen, daß selbst Wasserspuren, wie sie in der Luft enthalten sind, die gewünschte Umsetzung negativ beeinflussen bzw. verhindern.

Es muß daher als nicht vorhersehbar angesehen werden, daß die Umsetzung von Iminostilben mit einem Alkalicyanat in Essigsäuregemischen mit bis zu 20 Gewichtsprozent Wasser, bezogen auf die Gesamtlösungsmittelmenge, leicht und mit sehr guten bis guten Ausbeuten erfolgt.
Ebenso waren die erfindungsgemäß erhaltenen hohen Ausbeuten der Umsetzung in Essigsäure-Alkohol-Gemischen überraschend.
Es erweist sich gerade als Vorteil, die erfindungsgemäße Umsetzung in Essigsäuregemischen durchzuführen, wobei sehr gute Ausbeuten in Anwesenheit von 5 bis 10 % Wasser oder Alkohol in diesen Gemischen erhalten werden.

Das erfindungsgemäße Verfahren soll anhand der nachfolgenden Ausführungsbeispiele näher erläutert werden.

### Beispiel 1

Eine gerührte Suspension von 100 g Iminostilben in 1000 ml Essigsäure wird auf 60°C erwärmt. Danach wird innerhalb von 160 Minuten 54 g 90 %-iges Natriumcyanat in 11 Portionen zugegeben. Im Verlauf der Reaktion geht das Iminostilben fast vollständig in Lösung, bevor das Carbamazepin auszukristallisieren beginnt. Nach beendeter Zugabe des Natriumcyanates wird das Reaktionsgemisch weitere 20 Minuten bei 60°C gerührt.

Anschließend wird auf 18 - 20°C abgekühlt und 2 Stunden bei dieser Temperatur gerührt.
Das ausfallende Carbamazepin wird abgesaugt, mit 60 ml wasserfreier Essigsäure gewaschen und getrocknet.
Ausbeute: 107,8 g (87,9 % der Theorie), Fp.: 193 - 194°C Von der Mutterlauge wird im Wasserstrahlpumpenvakuum die Essigsäure abdestilliert. Aus dem verbliebenen Rückstand wird mit Wasser das Natriumacetat herausgelöst, das Carbamazepin abgesaugt, getrocknet und aus Toluol umkristallisiert. Dadurch erhält man weitere 9,8 (8,0%) g Carbamazepin mit einem Fp. von 191 - 193°C.
Das ergibt eine Gesamtausbeute von 95,9%.

### Beispiel 2

Eine Suspension von 100 g Iminostilben in einer Mischung aus 900 ml wasserfreier Essigsäure und 100 ml Wasser wird unter Rühren auf 60°C erwärmt. Danach wird innerhalb von 2,75 Stunden 58,3 g 90 %-iges Natriumcyanat in 16 Portionen zugegeben. Nach 2 Stunden Reaktionszeit wird kurzzeitig auf 80°C erhitzt, um geringe Mengen des ungelösten Iminostilben in Lösung zu bringen.
Nach beendeter Zugabe des Natriumcyanates wird das Reaktionsgemisch auf 18 - 20°C abgekühlt und 0,5 Stunden bei dieser Temperatur gerührt. Das ausfallende Carbamazepin wird abgesaugt, mit 70 ml eines Gemisches aus 63 ml Essigsäure und 9 ml Wasser gewaschen und getrocknet.
Ausbeute: 110,1 g (89,8 % der Theorie), Fp.: 191 - 195°C Von der Mutterlauge wird das Lösungsmittel abdestilliert. Aus dem verbliebenen Rückstand wird mit Wasser das Natriumacetat herausgelöst, das Carbamazepin abgesaugt, getrocknet und aus Toluol umkristallisiert.
Dadurch erhält man weitere 5 g (4,1%) Carbamazepin mit einem Fp. von 188 - 190°C.
Das ergibt eine Gesamtausbeute von 93,9%.

### Beispiel 3

Eine Suspension von 30 g Iminostilben in einer Mischung aus 225 ml Essigsäure und 45 ml Wasser wird unter Rühren auf 60°C erwärmt. Danach wird innerhalb von 2,75 Stunden 17,5 g 90 %-iges Natriumcyanat in 16 Portionen zugegeben. Nach 1,5 Stunden Reaktionszeit wird das Reaktionsgemisch kurzzeitig auf 80°C erhitzt, um geringe Mengen des ungelösten Iminostilben in Lösung zu bringen.
Nach beendeter Zugabe des Natriumcyanates wird das Reaktionsgemisch noch 10 Minuten bei 60°C gehalten. Danach wird auf 18 - 20°C abgekühlt und 1 Stunden bei dieser Temperatur gerührt. Das ausfallende Carbamazepin wird abgesaugt und mit einem 20 ml Essigsäure-Wasser-Gemisch (6 : 1) gewaschen und getrocknet.
Ausbeute: 32,9 g (89,2 % der Theorie), Fp.: 189 - 191°C Von der Mutterlauge wird das Lösungsmittel restlos abdestilliert. Aus dem verbliebenen Rückstand wird mit Wasser das Natriumacetat herausgelöst, das Carbamazepin abgesaugt, getrocknet und aus Toluol umkristallisiert.

Dadurch erhält man weitere 1,8 g (4,8%) Carbamazepin mit einem Fp. von 191 - 193°C. Das ergibt eine Gesamtausbeute von 94 %.

### Beispiel 4

Eine Suspension von 60 g Iminostilben in 600 ml Essigsäure wird unter Rühren auf 60°C erwärmt. Danach wird eine Lösung aus 40 g 98%igem Kaliumcyanat und 66 ml Wasser hergestellt und innerhalb von 2 Stunden zugetropft. Zwischenzeitlich erhitzt man das Reaktionsgemisch kurzzeitig auf 80°C, um ungelöstes Iminostilben in Lösung zu bringen.
Nach beendeter Zugabe des Kaliumcyanates wird das Reaktionsgemisch auf Raumtemperatur abgekühlt, das ausfallende Carbamazepin abgesaugt und getrocknet.
Es werden 33 g ( 89,9% der Theorie), Fp.: 190-193°C erhalten.
Die Mutterlaugenaufarbeitung analog dem Beispiel 3 ergibt weitere 3,3 g . Damit ergibt sich eine Gesamtausbeute von 93,2 % der Theorie.

### Beispiel 5

Eine gerührte Suspension von 100 g Iminostilben in einem Gemisch von 1000 ml Essigsäure und 150ml Wasser wird auf 60°C erwärmt. Danach wird innerhalb von 5 Stunden 60,8 g 98 %-iges Kaliumcyanat in 13 Portionen zugegeben.

Nach beendeter Zugabe des Kaliumcyanates wird das Reaktionsgemisch noch 30 Minuten gerührt und dann auf 18 - 20°C abgekühlt. Die ausgeschiedenen Kristalle werden abgesaugt, mit 60 ml Essigsäure und 400 ml Wasser gewaschen und getrocknet.
Ausbeute: 11,2 g (90,9 % der Theorie), Fp.: 191 - 193°C Von der Mutterlauge wird das Lösungsmittel abdestilliert und aus dem Rückstand das Kaliumacetat mit Wasser herausgelöst. Das Carbamazepin wird abgesaugt, mit Wasser gewaschen, aus Toluol umkristallisiert und getrocknet. Auf diese Weise werden weitere 7,5 g (6,1% d. Th.) Carbamazepin mit einem Fp. von 190 - 192°C erhalten.
Daraus ergibt sich eine Gesamtausbeute von 97 %.

### Beispiel 6

3 kg Iminostilben werden in einem Gemisch aus 28,5 l Essigsäure und 1,5 l Wasser unter Rühren auf 60°C erwärmt.
Innerhalb von ca 2 Stunden werden 1,66kg 98%iges Natriumcyanat zugegeben, es wird auf 15°C abgekühlt, der Ansatz noch 2 Stunden in einem Temperaturbereich von 20 bis 15°C gehalten, abgesaugt, mit 2 l Essigsäure gewaschen und getrocknet.
Ausbeute: 3,39 kg (92,5 % der Theorie), Fp.: 190-192°C Nach Abdestillieren von 22 l Essigsäure und Zugabe von 10 l Wasser zum Rückstand, kurzem Rühren, Absaugen und Waschen mit 5 l Wasser sowie Trocknen fallen weitere 0,28 kg Produkt an, die nach Umkristallisation aus Toluol 0,23 kg (6,3 % der Theorie) , Fp.: 191-194°C ergeben.
Die Gesamtausbeute beträgt 98,8 %.

### Beispiel 7

30 g Iminostilben werden in 360 ml Essigsäure und 50 ml Ethanol unter Rühren auf 60°C erwärmt und 20 g 98%iges Natriumcyanat innerhalb von 1,5 Stunden bei dieser Temperatur zugegeben. Man heizt kurz auf 80°C auf, rührt noch eine Stunde bei 60°C und kühlt auf 15°C , saugt ab, wäscht mit 20 ml Essigsäure und trocknet.
Ausbeute: 29,4 g ( 80,3% der Theorie), Fp.: 189-192°C.
Nach Zugabe von 1 g Natriumcyanat zur Mutterlauge, Eindestillieren der Mutterlauge, Zugabe von Wasser zum Rückstand und Umkristallisieren des Trockenproduktes aus Toluol erhält man weitere 4,9 g (13,4% der Theorie) Carbamazepin mit einem Schmelzpunkt von 190-193°C .
Die Gesamtausbeute beträgt 93,7 %.

## Patentansprüche

1. Verfahren zur Herstellung von 5H-Dibenz/b,f/azepin-5-carboxamid durch Umsetzung von Iminostilben mit Alkalicyanaten in Essigsäure, Essigsäure-Wasser- oder Essigsäure-Alkohol-Gemischen.

2. Verfahren gemäß Anspruch 1, wobei das Essigsäure-Wasser-Gemisch bis zu 20 Gewichtsprozent Wasser enthält.

3. Verfahren gemäß Anspruch 1 , wobei das Essigsäure-Alkohol-Gemisch bis zu 10 Gewichtsprozent Alkohol enthält.

4. Verfahren gemäß der Ansprüche 1 und 3 , wobei als Alkohole Methanol oder Ethanol verwendet werden.

5. Verfahren gemäß der Ansprüche 1 bis 4, wobei die Alkalicyanate allmählich zum Reaktionsgemisch zugegeben werden.

6. Verfahren gemäß der Ansprüche 1 bis 5 , wobei die Alkalicyanate in wäßriger Lösung zugegeben werden.

7. Verfahren gemäß der Ansprüche 1 bis 6, wobei als Alkalicyanate Natrium- oder Kaliumcyanat eingesetzt werden.

## Claims

1. Process for the preparation of 5H-dibenz[b,f]azepine-5-carboxamide by reaction of iminostilbene with alkali metal cyanates in acetic acid, acetic acid/water or acetic acid/alcohol mixtures.

2. Process according to Claim 1, the acetic acid/water mixture containing up to 20 per cent by weight of water.

3. Process according to Claim 1, the acetic acid/alcohol mixture containing up to 10 per cent by weight of alcohol.

4. Process according to one of Claims 1 and 3, the alcohols used being methanol or ethanol.

5. Process according to Claims 1 to 4, the alkali metal cyanates being added to the reaction mixture gradually.

6. Process according to Claims 1 to 5, the alkali metal cyanates being added in aqueous solution.

7. Process according to Claims 1 to 6, the alkali metal cyanates employed being sodium or potassium cyanate.

## Revendications

1. Procédé de préparation de 5H-dibenz/b,f/azépine-5-carboxamide par la réaction d'iminostilbène avec des cyanates alcalins dans l'acide acétique ou dans les mélanges acide acétique-eau ou acide acétique-alcool.

2. Procédé suivant la revendication 1, dans lequel le mélange acide acétique-eau contient jusqu'à 20% en poids d'eau.

3. Procédé suivant la revendication 1, dans lequel le mélange acide acétique-alcool contient jusqu'à 10% en poids d'alcool.

4. Procédé suivant les revendications 1 et 3, dans lequel les alcools utilisés sont le méthanol ou l'éthanol.

5. Procédé suivant les revendications 1 à 4, dans lequel les cyanates sont progressivement ajoutés au mélange réactionnel.

6. Procédé suivant les revendications 1 à 5, dans lequel les cyanates alcalins sont ajoutés en solution aqueuse.

7. Procédé suivant les revendications 1 à 6, dans lequel les cyanates alcalins utilisés sont le cyanate de sodium ou de potassium.
